# Europäisches Patentamt

# European Patent Office

# Office européen des brevets

(19)

(11) Numéro de publication: **0 296 082 B1**

(12) **FASCICULE DE BREVET EUROPEEN**

(45) Date de publication de fascicule du brevet: **27.04.94**   (51) Int. Cl.5: **A61K 39/395**

(21) Numéro de dépôt: **88430013.8**

(22) Date de dépôt: **06.06.88**

(54) **Agent actif et médicament en contenant destinés à prévenir ou combattre le rejet de greffe d'organe chez l'homme.**

(30) Priorité: **12.06.87 FR 8708320**

(43) Date de publication de la demande:
**21.12.88 Bulletin 88/51**

(45) Mention de la délivrance du brevet:
**27.04.94 Bulletin 94/17**

(84) Etats contractants désignés:
**AT BE CH DE ES FR GB GR IT LI LU NL SE**

(56) Documents cités:
**EP-A- 0 217 992
EP-A- 0 235 805
EP-A- 0 240 344**

**JOURNAL EXPERIMENTAL MEDICINE, vol. 162, juillet 1985, Rockefeller University Press; R.L. KIRKMAN et al., pp. 358-362&NUM;**

**TRANSPLANTATION PROCEEDINGS, vol. XIX, no. 1, février 1987, Grune & Stratton Inc.; M.E. SHAPIRO et al., pp. 594-598&NUM;**

**JOURNAL OF IMMUNOLOGY, vol. 136, no. 3, 01 février 1986, American Association of Immunologists, US; R.D. GRANSTEIN et al., pp. 898-902&NUM;**

(73) Titulaire: **IMMUNOTECH S.A.**
**70, route Léon Lachamp**
**Case 915- Luminy**
**F-13288 Marseille Cédex 9(FR)**

Titulaire: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Soulillou, Jean-Paul**
**32 ter rue de l'Abbaye**
**F-44100 Nantes(FR)**
Inventeur: **Peyronnet, Pierre**
**53 avenue Rudioux**
**F-87000 Limoges(FR)**
Inventeur: **Le Mauff, Brigitte**
**3 Impasse Baudelaire**
**F-44240 La Chapelle sur Erdre(FR)**
Inventeur: **Hourmant, Maryvonne**
**34 rue Emile Reder**
**F-44400 Rezé(FR)**
Inventeur: **Olive, Daniel**
**56 avenue Massenet**
**F-13009 Marseille(FR)**

Rank Xerox (UK) Business Services
(3.10/3.09/3.3.3)

PROCEEDINGS OF THE NATIONAL ACADEMY OF SCIENCES USA, vol. 83, avril 1986; J.W. KUPIEC-WEGLINSKI et al., pp. 2624-2627&NUM;

THE JOURNAL OF IMMUNOLOGY, vol. 136, no. 5, 01 mars 1986, American Association of Immunologists, US; Y. JACOUES et al., pp. 1693-1699&NUM;

NATURE, vol. 300, no. 5889, 18 novembre 1982, Chesham, Bucks (GB); W.J. LEONARD et al., pp. 267-269&NUM;

Inventeur: **Mawas, Claude**
**8, rue des Cinq Cents**
**F-13007 Marseille(FR)**
Inventeur: **Delaage, Michel**
**16 rue Adolphe Thiers**
**F-13001 Marseille(FR)**
Inventeur: **Hirn, Michel**
**Villa Gyptis**
**359 Corniche Kennedy**
**F-13008 Marseille(FR)**
Inventeur: **Jacques, Yannick**
**32bis rue Noire**
**F-44000 Nantes(FR)**

(74) Mandataire: **Santarelli, Marc et al**
**Cabinet Rinuy et Santarelli**
**14, avenue de la Grande Armée**
**F-75017 Paris (FR)**

**Description**

La présente invention concerne un agent actif destiné à prévenir ou à combattre le rejet de greffe d'organe chez l'homme ainsi qu'un médicament contenant un tel agent.

A l'heure actuelle, les greffes d'organes humains se développant de plus en plus, le besoin de disposer de moyens efficaces et fiables pour prévenir ou combattre le rejet de ces greffes d'organes prend une importance considérable pour favoriser la poursuite des travaux et des recherches dans le domaine chirurgical.

La présente invention fournit un tel moyen. Ce dernier est essentiellement constitué d'un anticorps monoclonal dirigé contre les récepteurs de l'interleukine 2 (IL2) humaine portés par les lymphocytes T activés, cet anticorps ayant en outre la propriété de ne pas être cytotoxique in vitro tout en inhibant la prolifération cellulaire induite par l'interleukine 2.

On sait que l'interleukine 2 est un facteur de croissance nécessaire pour le développment in vitro des clones de lymphocytes T (cf. MORGAN D.A., RUSCETTI F.W., GALLO R., Selective in vitro growth of T lymphocyte from normal human bone marrow . Science 1976 ; 193 : 1007-11 (1) ), qui agit par interaction avec un récepteur spécifique à forte affinité (cf. ROBB R.J., MUNCK A., SMITH K.A., T-cell growth factor receptors. Quantitation specificity and biological relevance. J. Exp. Med. 1981, 154 ; 1455-67 (2) ). L'expression du récepteur de l'IL2 (R-IL2) à la surface de la membrane du lymphocyte T est sous le contrôle de l'activation antigénique et est soumis à une régulation supplémentaire par l'IL2 elle-même (cf. JACQUES Y., LE MAUFF B., GODARD A., OLIVE D., MOREAU J.F., SOULILLOU J.P., Regulation of Interleukine 2 receptor expression on a human cytotoxic T lymphocyte clone, synergism between alloantigenic stimulation and IL2., J. Immunol. 1986 ; 136 : 1693 - 99 (3) ).

Le R-IL2 se révèle être un complexe formé d'une chaîne (55 Kd) (antigène Tac) et d'une chaîne $\beta$ récemment décrite (75 Kd), les deux chaînes étant nécessaires pour l'expression du site à forte affinité (cf. SHARON M.; KLAUSNER R.P., CULLEN B.R., CHIZZONITE R., LEONARD W.J., Novel Interleukin-2, Receptor subunit detected by cross-linking under high affinity conditions, Science 1986 ; 134 ; 859-63 (4)).

On sait aussi que des anticorps monoclonaux spécifiques des R-IL2 de rongeurs et capables de supprimer la prolifération in vitro des lymphocytes T activés, induite par l'IL2, se sont révélés empêcher ou inverser le processus de rejet aigu d'une allogreffe cardiaque chez la souris (cf. KIRKMAN R.L., BARRET L.V., GAULTON G.N., KELLEY V., YTHIER A., STROM T.B., Administration of an anti-interleukin 2 receptor monoclonal antibody prolongs cardiac allograft survival in mice, J. Exp. Med., 1985 ; 162 : 358-62 (5)), et retarder le rejet de peau chez la souris (cf. GRANDSTEIN R.D., GOULSTON C., GAULTON G.N., Prolongation of murine skin allograft survival by immunologic manipulation with anti-interleukin 2 receptor antibody, J. Immunol. 1986, 136 : 898-902 (6)) ou le rejet de rein chez le singe (cf. SHAPIRO M.E., KIRKMAN R.L., REED M.H., PUSKAS J.P., MAZOUJIAN G., LETVIN N.L., CARPENTER C.B., MILFORD E.L., WALDMAN T.A., STROM T.B., SCHLOSSMAN S.F., Monoclonal anti-IL2 receptor antibody in primate renal transplantation, Transplt. Proc. 1987 : 594-98 (7)). De même, chez l'homme, plusieurs anti-R-IL2 ont été obtenus à partir de la souris (cf. UCHIYAMA T., BRODER S., WALDMAN T.A., A monoclonal antibody (anti-Tac) reactive with activated and functionally mature human T cells, J. Immunol. 1981 ; 126 : 1383-97 (8)) et du rat (cf. OLIVE D., RAYMOND J., DUBREUIL P., CHARMOT D., JACQUES Y., MAWAS C., Anti Interleukin 2 receptor monoclonal antibody-receptor interactions in their antagonistic effects on interleukin-2-dependent T-cell growth, Eur. J. Immunol. 1986 ; 16 : 611-20 (9)), tous reconnaissant le constituant 55 Kd du R-IL2. Des travaux récents ont montré que les répertoires immunologiques du rat et de la souris distinguent deux épitopes principaux sur le R-IL2 humain. Un de ceux-ci, étroitement apparenté au site d'interaction avec l'IL2, provoque un blocage fonctionnel ; l'autre est indépendant du site de liaison à l'IL2 et il est incapable d'inhiber la prolifération des lymphocytes T activés induite par l'IL2.

Or, les Demandeurs ont constaté de façon inattendue que des anticorps ayant justement la propriété de ne pas être cytotoxiques, même en présence de complément, tout en inhibant la prolifération des cellules induite par l'interleukine 2 étaient bien tolérés par des patients ayant subi la greffe d'un organe et que ces anticorps étaient efficaces pour empêcher le rejet de cet organe greffé et pouvaient donc entrer dans la composition de médicaments et notamment l'anticorps monoclonal de rat 33 B 3.1 décrit par Olive et al dans Eur. J. Immmunol. 1986, 16, 611-20 (9).

C'est pourquoi la présente invention à pour objet :

Un anticorps monoclonal caractérisé en ce qu'il est capable de se fixer au récepteur de l'IL2 humain porté par les lymphocytes et en ce qu'il n'est pas cytotoxique in vitro, même en présence de complément, tout en inhibant la prolifération cellulaire induite par l'IL2, pour son utilisation dans une méthode de traitement thérapeutique du corps humain. On citera en particulier comme anticorps selon l'invention, un anticorps monoclonal de souris de classe IgG1 ; un anticorps monoclonal de rat de classe IgG2a ; un anticorps monoclonal humain ; un anticorps monoclonal chimère comportant une partie d'origine humaine et une partie d'origine animale ; un anticorps d'origine humaine ou animale modifié chimiquement (par exemple au moyen de polyéthylèneglycol) pour supprimer les effets cytotoxiques en présence de complément.

La présente invention a aussi pour objet les compositions pharmaceutiques caractérisées en ce qu'elles sont constituées d'un anticorps tel que défini ci-dessus ainsi que d'un excipient pharmaceutiquement acceptable.

La présente invention a enfin pour objet un anticorps monoclonal tel que défini ci-dessus pour son utilisation comme agent de lutte conte le rejet de greffe d'organe.

D'autres caractéristiques et les avantages de l'invention ressortiront plus clairement de la description qui va suivre, faite en prenant comme exemple d'anticorps selon l'invention, l'anticorps connu sous l'appellation 33B3.1 qui est un anticorps monoclonal (AcMo) du type IgG2a provenant du rat.

Cet anticorps est produit à partir de liquide d'ascite de souris "nude" auxquelles on a injecté les cellules de l'hybridome 33B3.1. Les cellules et l'anticorps purifié ont été vérifiés pour l'absence de virus murins et de mycoplasmes. Pour les expérimentations et les essais qui seront relatés ci-après, l'anticorps en question se présentait en flacons de 5 ml contenant 5 mg de 33B3.1 dans 1 ml de sérum physiologique tamponné par un phosphate. Ces flacons étaient maintenus à 4°C jusqu'à leur utilisation. Le but de ces expérimentations et de ces essais était de montrer l'effet immunosuppresseur du 33B3.1 administré à titre de traitement préventif du rejet en transplantation rénale.

Exemple d'expérimentation

1) Protocole :

L'AcMo 33B3.1 a été administré quotidennement pendant 14 jours, en plus de 1 mg/kg de prednisone (CS) au jour 0, dose diminuée de 10 mg/semaine et avec 2 mg/kg d'azathioprine (AZA). L'AcMo, dilué dans 100 ml de sérum physiologique, a été injecté quotidiennement par voie intraveineuse (veine périphérique, injection intraveineuse lente pendant 10 minutes). L'administration de cyclosporine A (CyA) a commencé (6 mg/kg) à la fin du traitement par le 33B3.1 Des GAT (Thymoglobuline, Institut Mérieux, Lyon, France) ont été administrées à titre de traitement de secours de sept jours. Ainsi, dans le cas du rejet prouvé par biopsie au cours d'un traitement par l'AcMo, l'administration de 33B3.1 a été interrompue et l'administration de GAT a commencé à 2 mg/kg/jour et a été ensuite adaptée au maintien d'une teneur en lymphocytes T11$^+$ circulants inférieure à 10 %.

Par ailleurs, des biopsies percutanées de rein ont été effectuées systématiquement au jour 9. Une fois l'administration de CyA commencée, les administrations de CS et d'AZA ont été progressivement diminuées et finalement supprimées en trois semaines. Chez les patients sous CyA, les épisodes de rejets ont été traités par des injections intraveineuses lentes de méthylprednisolone pendant 5 jours (respectivement 5, 5, 4, 3 et 2 mg/kg/jour), puis par une dose orale de 1,5 mg/kg/jour de stéroïdes, dose qui a été diminuée lentement jusqu'à l'arrêt.

2) Patients :

Ils étaient au nombre de vingt-sept, dont le consentement a été obtenu après les avoir pleinement informés. Tous étaient receveurs d'une greffe primaire provenant de cadavre et possédaient des alloanticorps réagissant avec moins de 50 % d'un ensemble de lymphocytes. Tous les patients ont été préalablement soumis à une transfusion et les greffes familiales ont été exclues. Neuf sujets ont reçu 5 mg/jour de 33B3.1 (groupe A) et 18 autres patients 10 mg/jour (groupe B). Les examens de contrôle ont duré du 35e au 345e jour. Ces deux groupes de receveurs ont été comparés à :
- Un groupe "témoin historique" de 30 receveurs (groupe C) ayant subi une greffe en 1981. Ces patients n'ont reçu qu'un CS (2 mg/kg/jour pendant deux semaines, dose abaissée lentement à 15 mg/jour à la fin du deuxième mois) et de l'AZA (2 mg/kg et suivant leur formule leucocytaire - FL) ; et
- Un groupe de 55 receveurs (groupe D) ayant reçu un traitement similaire à celui du groupe A ou B, sauf que les GAT ont été administrées (fistule artério-veineuse brachiale ou cathéter central) à la place de 33B3.1.

Les receveurs des groupes C et D ont été également soumis à des transplantations primaires à partir de cadavres, sans hyperimmunisation (le tableau I ci-après présente leurs caractéristiques cliniques et immunologiques générales).

**Tableau I** : Caractéristiques cliniques et immunologiques
des différents groupes receveurs

| | GROUPE A 33B3.1 5 mg/j + Cs + AZA (n=9) | GROUPE B 33B3.1 10 mg/j + Cs + AZA (n=18) | GROUPE C Cs + AZA (n=30) | GROUPE D GAT + CS + AZA (n=55) |
|---|---|---|---|---|
| Age* | 35(11-53) | 37(23-61) | 30(10-49) | 35(10-61) |
| Sex-ratio M/F | 3,5 | 5 | 1,5 | 1,6 |
| Nombre de transfusions* | 5(2-12) | 5(2->50) | 5(2-22) | 6(2-25) |
| PATIENTS avec 3 ou moins de 3 antigènes H L A A-B-DR mal appariés | 22 % | 66 % | 73 % | 58 % |
| Réactivité globale *** | | | | |
| . Lymphocytes T | <20% n=8 >20% n=1 | 14 4 | 27 3 | 45 10 |
| . Lymphocytes B | <20% n=7 >20% n=2 | 14 4 | ** ** | 34 21 |
| Ischémie froide * durée (heures) | 35(18-48) | 34,5(21-48) | 21,5(9-29) | 34,5(6-50) |

```
  * Moyenne et valeurs extrêmes
 ** Résultats non disponibles
*** Tous les patients présentaient une réactivité globale inférieure
      à 50 %
```

3) Teneurs circulantes en 33B3.1 et contrôle de la réponse immunitaire anti-33B3.1 des receveurs :

Teneurs sanguines en 33B3.1 :

Les teneurs sanguines ont été mesurées par ELISA sur des sérums obtenus avant et après la greffe à intervalles réguliers. Les plaques d'ELISA ont été revêtues de Mark I, un Ac de souris antichaîne légère Kappa de rat, et la présence de 33B3.1 dans les sérums a été révélée avec de l'anti-Fab'$_2$ de rat (marqué à la $\beta$-galactosidase) provenant du mouton. Les concentrations sériques en 33B3.1 ont été déterminées d'après une courbe d'étalonnage classique obtenue avec des dilutions successives de 33B3.1 pur.

Détection de l'anti - 33B3.1 :

La présence d'IgG ou d'IgM anti-33B3.1 dans les sérums recueillis pendant et après le traitement à l'AcMo a été testée par ELISA. Des plaques ont été revêtues de 33B3.1 pur, mises à incuber avec des sérums dilués des receveurs et révélées avec une anti-IgM ou IgG humaine marquée avec une peroxydase provenant de la chèvre. La réaction a été considérée come étant positive lorsque la valeur moyenne de la double détermination était supérieure de trois écarts-types à la valeur moyenne témoin.

Résultats

1) Tolérance au traitement :

Le 33B3.1 a été bien toléré. Seuls cinq patients sur vingt sept ont présenté des effets secondaires minimes : l'un, éruption cutanée, deux autres des douleurs veineuses locales, un autre un vertige et une réaction anaphylactoïde lors de la dernière injection intraveineuse lente d'AcMo. En aucun cas, ces effets secondaires n'ont conduit à l'interruption du traitement. Une fièvre d'intensité faible a été habituellement observée au cours des premières 48 heures. A l'opposé, une fièvre de forte intensité a été observée chez 52 % des patients du groupe D, et respectivement 18 et 25 % d'entre eux présentaient une éruption cutanée et une maladie sérique aigüe.

Aucune différence (ni diminution) des numérations d'hématies et de plaquettes n'a été notée entre les patients des groupes A, B et C suggérant que le 33B3.1 possède seulement une incidence faible sur ces paramètres. Le nombre de cellules polynucléaires a été multiplié par 1,7 à 2 au cours du traitement par l'AcMo. Une faible lymphopénie a été observée (diminution de 20 % des valeurs de départ, culminant au jour 6), qui contrastait néanmoins avec la chute brutale des comptages de lymphocytes (P < 0,01 test t de Fisher) observée dans le groupe D. Les rapports $T4^+$ / $T8^+$ n'ont pas été modifiés chez les patients des groupes A et B pendant tout le traitement par le 33B3.1. Deux patients du groupe A et quatre du groupe B ont présenté des épisodes d'infection virale (5 CMV et 1 EVB). Un patient du groupe A a présenté une candidose buccale étendue.

2) Incidence du rejet :

Jusqu'à présent, pour les groupes A et B réunis, tous les patients, sauf deux, possèdent un rein fonctionnel : un s'est suicidé (jour 32, histologie du rein normale) et un autre a présenté une rupture technique (élimination de la greffe par nécrose urétérale profonde, histologie du rein normale). Parmi les neuf patients du groupe A, trois ont présenté des symptômes de rejet pendant la période d'injection intraveineuse lente de 33B3.1 (tableau II ci-après).

Tableau II

| Incidence d'un rejet aigu chez les divers groupes au cours du premier mois suivant la transplantation | | | | |
|---|---|---|---|---|
| | GROUPE A 33B3.1 5mg/j (n = 9) | GROUPE B 33B3.1 10 mg/j (n = 18) | GROUPE C Témoin historique (n = 30) | GROUPE D GAT (n = 55) |
| Rejet au cours de la période de traitement par l'AcMo ou d'une période similaire (jours 0 à 13) | 33,3 % | 5,6 %* | 66,6 % | 4 % |
| Rejet au cours du premier mois | 44,4 % | 5,6 %** | 76,6 % | 15 % |

\* p < 0.001, comparativement au groupe C
\*\* un patient, présentant un échec technique, non pris en considération

Dans tous les cas, des biopsies rénales ont confirmé le diagnostic. Les épisodes de rejet étaient tous réversibles après un traitement de secours. Parmi les 18 patients du groupe B, un seul a présenté un épisode de rejet, réversible au moyen du traitement de secours. Néanmoins, des biopsies systématiques, effectuées au jour 9 (4 patients du groupe A et 5 du groupe B) ont montré un certain degré d'infiltration des lymphocytes en l'absence de symptômes cliniques de rejet, pouvant être compatible avec un "rejet histologique" faible. Bien que ces patients n'aient reçu aucun traitement de secours par GAT, leur fonction rénale est restée intacte et aucun rejet "clinique" ne s'est produit. A l'opposé, 66 % des patients du groupe témoin historique ont présenté un rejet aigu dans les deux premières semaines (P > 0,001, comparativement au groupe B, test $\chi^2$). Quatre pour cent des receveurs sous GAT ont présenté un épisode de rejet au cours d'une période similaire. Réunis, ces résultats suggèrent un effet protecteur fort du 33B3.1 utilisé à 10 mg/jour, contre un rejet précoce.

En outre, deux patients (tous deux n'ayant présenté aucun rejet au cours du traitement par 33B3.1) ont présenté un épisode de rejet après la période d'injection intraveineuse lente d'AcMo : au jour 47 (groupe A) et au jour 35 (groupe B).

3) Teneurs sanguines en AcMo et réponse immunitaire anti-33B3.1 des receveurs

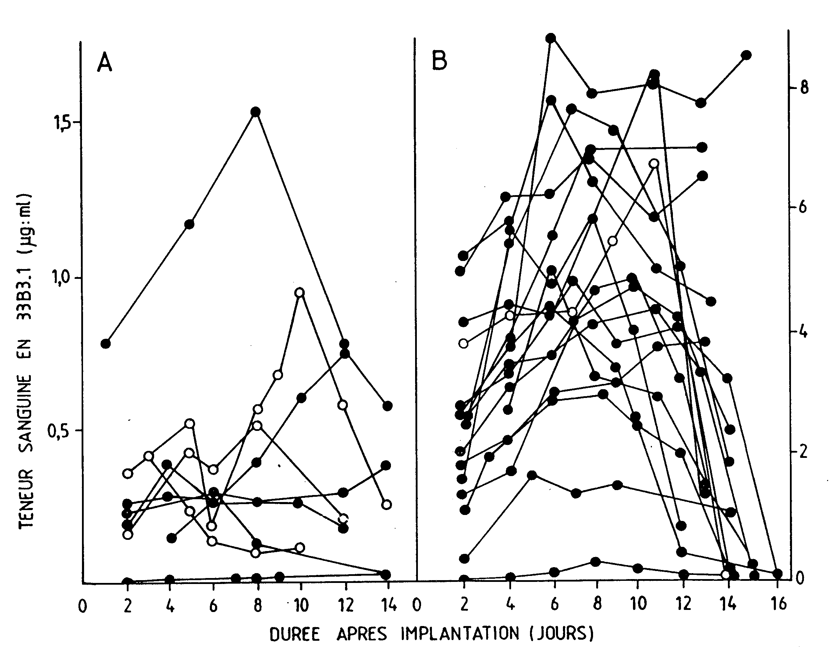

La figure année montre les teneurs sanguines (24 heures après injection intraveineuse lente) en 33B3. 1 circulant. Dans le groupe A, un patient (n'ayant jamais présenté de rejet) possédait une teneur en 33B3.1 circulant indétectable et les autres (comprenant ceux présentant un rejet pendant le traitement par le 33B3.1 ) présentaient une teneur sanguine allant d'une valeur indétectable à 1,6 μg/ml. Les receveurs du groupe a présentaient sans ambiguïté une teneur circulante supérieure (1,5 à 9 μg/ml), y compris les seuls receveurs de ce groupe ayant présenté un rejet. Cependant, un patient possédait encore une teneur basale faible (0,3 μg/ml).

Le tableau III ci-après montre que, parmi les 27 patients, 23 ont produit des anticorps contre le 33B3.1, d'isotype IgM (20/27) et/ou IgG (21/27). Seize patients possédaient des anticorps anti-33B3.1 détectables au cours du traitement par l'AcMo, sans rejet. La réponse des IgM et IgG a atteint un pic environ 10 jours après la fin du traitement par le 33B3.1.

Quant à la figure annexée qui donne les teneurs sanguines en 33B3.1, mesurées au cours du traitement par l'AcMo, les diagrammes A et B correspondent respectivement aux valeurs obtenues dans les groupes A et B avec -O-désignant des patients ayant présenté des épisodes de rejet et -●-ceux n'ayant présenté aucun rejet.

De l'exemple ci-dessus, il ressort qu'un AcMo IgG2a de rat dirigé contre le site de liaison de l'IL2 sur le R-IL2 humain était capable d'empêcher le rejet aigu précoce d'un greffon. Cet AcMo était très bien toléré ; seuls des effets secondaires rares et de faible importance ont été observés. Dans un premier groupe dans lequel l'AcMo a été administré à 5 mg/jour, trois receveurs sur neuf ont présenté un épisode de rejet (réversible avec un traitement de secours par GAT) lorsqu'ils étaient sous traitement par 33B3.1. Des considérations théoriques suggérant que cette première dose de 33B3.1 peut être trop faible pour présenter

Tableau III : Réponse anti-33B3.1 des patients des groupes A et B

| | Réponse par IgM | | | Réponse par IgG | | |
|---|---|---|---|---|---|---|
| | Nombre de patients positifs | Temps nécessaire (en jours) pour les teneurs maximales | Nombre de patients avec des IgM au cours du traitement par AcMo | Nombre de patients positifs | Temps nécessaire (en jours) pour les teneurs maximales | Nombre de patients avec des IgG au cours du traitement par AcMo |
| Groupe A n=9 | 6 | $23 \pm 5$ | 1 | 7 | $24 \pm 8$ | 5 |
| Groupe B n=18 | 14 | $21 \pm 4$ | 10 | 14 | $24 \pm 7$ | 8 |

un effet optimal, on a commencé le traitement d'une seconde série à 10 mg/jour, conduisant à une protection efficace. Dans ce groupe, un seul patient sur 18, a présenté des symptômes de rejet cliniquement détectables au cours des deux premières semaines suivant la transplantation (période de traitement par l'AcMo), comparativement à l'incidence de 66 % d'épisodes de rejet notée pendant la même période chez le groupe "témoin historique" ($P < 0,001$). Bien que des transplantations anciennes aient été effectuées chez les patients de ce dernier groupe, le plan de transfusion et la stratégie d'appariement étaient similaires. Evidemment, si la concordance des HLA était faible chez le petit groupe A, les groupes B, C ou D possédaient une concordance similaire. De plus, les patients du groupe témoin historique ont reçu une posologie initiale de stéroïdes double de celle des patients des groupes A et B, comme le montre le tableau II ; avec les précautions nécessaires dues à la taille limitée de l'échantillon, le pourcentage de rejet observé chez les patients recevant 10 mg/jour de 33B3.1 était sensiblement similaire à celui observé chez les patients du groupe D auxquels une GAT, thérapeutique anti-rejet bien établie et efficace, a été administrée à la place du 33B3.1. Deux patients ayant reçu l'AcMo ont présenté des épisodes de rejet au bout des deux premières semaines, ce qui suggère qu'aucune délétion de clones ($R^+$-IL2) n'a eu lieu chez les patients traités par le 33B3.1 et que les rejets en retour ont été rares.

Aucun épisode infectieux grave ne s'est produit parmi les patients du groupe A ou B.

Ainsi, les patients sous 33B3.1 n'ont pas présenté la lymphopénie profonde observée chez les patients du groupe D. Les lymphocytes $T11^+$, $T4^+$ et $T8^+$ ont diminué légèrement et harmonieusement (c'est-à-dire rapports T4/T8 similaires) au cours du traitement par l'AcMo, conformément au fait que les deux sous-ensembles expriment les R-IL2 au cours du rejet (cf. ci-dessus) et que les lymphocytes activés restent en minorité parmi les leucocytes périphériques. Tous les comptages de globules blancs ont notablement augmenté, comparativement au groupe D. Toutes ces variations concernant les leucocytes traduisent probablement l'association de l'effet du CS et de l'absence de réaction croisée néfaste du 33B3.1.

Le contrôle des teneurs en 33B3.1 a révélé qu'un patient du groupe A ne possédait aucune teneur sanguine détectable (bien que ne présentant aucun rejet), tandis que tous les patients du groupe B possédaient une teneur sanguine élevée et vraisemblablement saturante en 33B3.1 (conformément à l'affinité de l'AcMo pour le R-IL2) pouvant expliquer l'incidence de rejet très faible observée dans le groupe B. La plupart des patients ont produit des anticorps contre le 33B3.1. De même, dans cette petite série, aucune corrélation n'a pu être trouvée entre la manifestation d'un rejet (groupe A ou B) et la détection d'anticorps IgG et/ou IgM anti-33B3.1. Des tentatives pour estimer la présence d'anticorps anti-idiotypes ont toujours échoué en raison de concentrations contaminantes détectables de R-IL2 solubles. Chez certains patients (tableau III), il a été observé de manière inattendue des anticorps anti-33B3.1 dès le dixème jour après transplantation, ce qui était plus précoce que ce qui a été observé chez des receveurs traités par l'OKT3 (cf. GOLDSTEIN G., FUCELLO A.J., NORMAN P.J., SHIELD C.F., COLVIN R.B., COSINI B., OKT3 monoclonal antibody plasma levels during therapy and the subsequent development of host antibodies to OKT3, Transplantation 1986 ; 42 : 507-10 (12) et cf. CHATENOUD L., BAUDRIHAYE M.F., CHKOFF N., KREIS H., GOLDSTEIN G., BACH J.F., The restriction of the human in vivo immune response against the mouse monoclonal antibody OKT3, J. Immunol. 1986 ; 137 : 830-38 (13)).

Ce nouveau traitement, utilisant un AcMo dirigé contre un récepteur de facteur de croissance présent seulement sur les lymphocytes T activés, médicalement bien toléré et efficace dans la prévention d'un épisode de rejet précoce, présente ainsi un intérêt primordial dans la modulation de la réponse allogénique chez l'homme. Il est donc applicable à n'importe quel type de greffe, non seulement le rein, mais aussi le coeur, le foie, le poumon, l'intestin, la moelle osseuse, etc...

Du fait que l'anticorps agit par simple compétition avec l'interleukine 2, il est naturellement remplaçable par tout autre anticorps monoclonal de spécificité comparable de souris ou d'home. Egalement, on doit considérer qu'un anticorps chimère combinant un site actif bloquant le récepteur de l'interleukine 2, apporté par un anticorps d'origine murine et une région constante d'origine humaine, sera également convenable.

L'important est de bloquer la fonction du récepteur sans tuer la cellule, ce qui évite tous les symtômes d'intoxication (le syndrome des brûlés) observés avec les anticorps cytotoxiques.

Enfin, il est possible d'utiliser un anticorps quelconque dont on aurait fait disparaître la cytotoxicité par greffage de groupements chimiques, et notamment de polyéthylèneglycol. Toutes ces substitutions qui respectent le concept d'anticorps bloquant les effets de l'interleukine 2 sans tuer les lymphocytes sont partie intégrante de l'invention.

**Revendications**
**Revendications pour les Etats contractants suivants : AT, BE, CH, DE, FR, GB, IT, LI, LU, NL, SE**

**1.** Anticorps monoclonal caractérisé en ce qu'il est capable de se fixer au récepteur de l'$IL_2$ humain porté par les lymphocytes et en ce qu'il n'est pas cytotoxique in vitro, même en présence de complément, tout en inhibant la prolifération cellulaire induite par l'$IL_2$, pour son utilisation dans une méthode de traitement thérapeutique du corps humain.

**2.** Anticorps selon la revendication 1, caractérisé en ce que ledit anticorps est un anticorps monoclonal de souris de classe IgG1.

**3.** Anticorps selon la revendication 1, caractérisé en ce que ledit anticorps est un anticorps monoclonal de rat de classe IgG2a.

**4.** Anticorps selon la revendication 1, caractérisé en ce que ledit anticorps est un anticorps monoclonal humain.

**5.** Anticorps selon la revendication 1, caractérisé en ce que ledit anticorps est un anticorps monoclonal d'origine humaine ou animale modifié chimiquement pour supprimer les effets cytotoxiques en présence de complément.

**6.** Anticorps selon la revendication 1, caractérisé en ce que ledit anticorps d'origine humaine ou animale est chimiquement modifié au moyen de polyéthylèneglycol.

**7.** Compositions pharmaceutiques caractérisées en ce qu'elles sont constituées d'un anticorps selon l'une quelconque des revendications 1 à 6 à titre de principe actif ainsi que d'un excipient pharmaceutiquement acceptable.

**8.** Compositions pharmaceutiques caractérisées en ce qu'elles sont constituées d'un anticorps selon la revendication 3 à titre de principe actif ainsi que d'un excipient pharmaceutiquement acceptable.

**9.** Anticorps monoclonal tel que défini à la revendication 1 pour son utilisation comme agent de lutte contre le rejet de greffe d'organes.

**10.** Anticorps monoclonal tel que défini à l'une quelconque des revendications 2 et 4 à 6 pour son utilisation comme agent de lutte contre le rejet de greffe d'organes.

**11.** Anticorps monoclonal tel que défini à la revendication 3 pour son utilisation comme agent de lutte contre le rejet de greffe d'organes.

**Revendications pour les Etats contractants suivants : ES, GR**

**1.** Procédé de préparation d'une composition pharmaceutique, caractérisé en ce que l'on mélange un anticorps monoclonal capable de se fixer au récepteur de l'$IL_2$ humain porté par les lymphocytes et n'étant pas cytotoxique in vitro, même en présence de complément, tout en inhibant la prolifération cellulaire induite par l'$IL_2$, avec un excipient pharmaceutiquement acceptable.

**2.** Procédé selon la revendication 1, caractérisé en ce que ledit anticorps est un anticorps monoclonal de souris de classe IgG1.

**3.** Procédé selon la revendication 1, caractérisé en ce que ledit anticorps est un anticorps monoclonal de rat de classe IgG2a.

**4.** Procédé selon la revendication 1, caractérisé en ce que ledit anticorps est un anticorps monoclonal humain.

**5.** Procédé selon la revendication 1, caractérisé on ce que ledit anticorps est un anticorps monoclonal d'origine humaine ou animale modifié chimiquement pour supprimer les effets cytotoxiques en

EP 0 296 082 B1

présence de complément.

6. Procédé selon la revendication 1, caractérisé en ce que ledit anticorps d'origine humaine ou animale est chimiquement modifié au moyen de polyéthylèneglycol.

7. Utilisation d'un anticorps monoclonal capable de se fixer au récepteur de l'IL$_2$ humain porté par les lymphocytes et n'étant pas cytotoxique in vitro, même en présence de complément, tout en inhibant la prolifération cellulaire induite par l'IL$_2$ pour préparer un médicament destiné à la lutte contre le rejet de greffe d'organes.

8. Utilisation d'un anticorps monoclonal tel que défini à la revendication 3 pour préparer un médicament destiné à la lutte contre le rejet de greffe d'organes.

9. Utilisation d'un anticorps monoclonal tel que défini à l'une quelconque des revendications 2 et 4 à 6 pour préparer un médicament destiné à la lutte contre le rejet de greffe d'organes.

**Claims**
**Claims for the following Contracting States : AT, BE, CH, DE, FR, GB, IT, LI, LU, NL, SE**

1. Monoclonal antibody characterized in that it is capable of being fixed on the human IL$_2$ receptor carried by lymphocytes and in that it is not cytotoxic in vitro, even in the presence of complement, while inhibiting the cell proliferation induced by IL$_2$, for its use in a therapeutic treatment method of the human body.

2. Antibody according to claim 1, characterized in that the said antibody is a mouse monoclonal antibody of class IgG1.

3. Antibody according to claim 1, characterized in that the said antibody is a rat monoclonal antibody of class IgG2a.

4. Antibody according to claim 1, characterized in that the said antibody is a human monoclonal antibody.

5. Antibody according to claim 1, characterized in that the said antibody is a monoclonal antibody of human or animal origin chemically modified in order to suppress the cytotoxic effects in the presence of complement.

6. Antibody according to claim 1, characterized in that the said antibody of human or animal origin is chemically modified by means of polyethyleneglycol.

7. Pharmaceutical compositions characterized in that they are constituted by an antibody according to any one of claims 1 to 6 as active ingredient as well as by a pharmaceutically acceptable excipient.

8. Pharmaceutical compositions characterized in that they are constituted by an antibody according to claim 3 as active ingredient as well as by a pharmaceutically acceptable excipient.

9. Monoclonal antibody as defined in claim 1 for its use as an agent for combating the rejection of organ transplants.

10. Monoclonal antibody as defined in any one of claims 2 and 4 to 6 for its use as an agent for combating the rejection of organ transplants.

11. Monoclonal antibody as defined in claim 3 for its use as an agent for combating the rejection of organ transplants.

**Claims for the following Contracting States : ES, GR**

1. Preparation process for a pharmaceutical composition, characterized in that a monoclonal antibody capable of being fixed to the human IL$_2$ receptor carried by the lymphocytes and not being cytotoxic in

11

EP 0 296 082 B1

vitro, even in the presence of complement, while inhibiting the cell proliferation induced by $IL_2$, is mixed with a pharmaceutically acceptable. excipient.

2. Process according to claim 1, characterized in that the said antibody is a mouse monoclonal antibody of class IgG1.

3. Process according to claim 1, characterized in that the said antibody is a rat monoclonal antibody of class IgG2a.

4. Process according to claim 1, characterized in that the said antibody is a human monoclonal antibody.

5. Process according to claim 1, characterized in that the said antibody is a monoclonal antibody of human or animal origin chemically modified in order to suppress the cytotoxic effects in the presence of complement.

6. Process according to claim 1, characterized in that the said antibody of human or animal origin is chemically modified by means of polyethyleneglycol.

7. Use of a monoclonal antibody capable of being fixed to the human $IL_2$ receptor carried by the lymphocytes and not being cytotoxic in vitro, even in the presence of complement, while inhibiting the cell proliferation induced by $IL_2$ for preparing a medicament intended for combating the rejection of organ transplants.

8. Use of a monoclonal antibody as defined in claim 3 for preparing a medicament intended for combating the rejection of organ transplants.

9. Use of a monoclonal antibody as defined in any one of claims 2 and 4 to 6 for preparing a medicament intended for combating the rejection of organ transplants.

**Patentansprüche**
**Patentansprüche für folgende Vertragsstaaten : AT, BE, CH, DE, FR, GB, IT, LI, LU, NL, SE**

1. Monoklonaler Antikörper, dadurch gekennzeichnet, daß er an den auf den Lymphozyten befindlichen menschlichen $IL_2$-Rezeptor binden kann und daß er in vitro, auch in Gegenwart von Komplement, nicht zytotoxisch ist, dabei aber die durch $IL_2$ induzierte Zellvermehrung inhibiert, zur Verwendung bei einer therapeutischen Behandlungsmethode des menschlichen Körpers.

2. Antikörper nach Anspruch 1, dadurch gekennzeichnet, daß der Antikörper ein monoklonaler Antikörper der Maus der Klasse IgG1 ist.

3. Antikörper nach Anspruch 1, dadurch gekennzeichnet, daß der Antikörper ein monoklonaler Antikörper der Ratte der Klasse IgG2a ist.

4. Antikörper nach Anspruch 1, dadurch gekennzeichnet, daß der Antikörper ein menschlicher monoklonaler Antikörper ist.

5. Antikörper nach Anspruch 1, dadurch gekennzeichnet, daß der Antikörper ein monoklonaler Antikörper menschlichen oder tierischen Ursprungs ist, der chemisch modifiziert wurde, um die zytotoxischen Wirkungen in Gegenwart von Komplement zu unterdrücken.

6. Antikörper nach Anspruch 1, dadurch gekennzeichnet, daß der Antikörper menschlichen oder tierischen Ursprungs mit Polyethylenglycol chemisch modifiziert ist.

7. Pharmazeutische Zusammensetzungen, dadurch gekennzeichnet, daß sie durch einen Antikörper nach einem der Ansprüche 1 bis 6 als Wirkstoff sowie durch einen pharmazeutisch akzeptablen Exzipienten gebildet sind.

12

EP 0 296 082 B1

**8.** Pharmazeutische Zusammensetzungen, dadurch gekennzeichnet, daß sie durch einen Antikörper nach Anspruch 3 als Wirkstoff sowie durch einen pharmazeutisch akzeptablen Exzipienten gebildet sind.

**9.** Monoklonaler Antikörper nach Anspruch 1 zur Verwendung als Mittel zur Bekämpfung der Abstoßung von Organtransplantaten.

**10.** Monoklonaler Antikörper nach einem der Ansprüche 2 und 4 bis 6 zur Verwendung als Mittel zur Bekämpfung der Abstoßung von Organtransplantaten.

**11.** Monoklonaler Antikörper nach Anspruch 3 zur Verwendung als Mittel zur Bekämpfung der Abstoßung von Organtransplantaten.

**Patentansprüche für folgende Vertragsstaaten : ES, GR**

**1.** Verfahren zur Herstellung einer pharmazeutischen Zusammensetzung, dadurch gekennzeichnet, daß man einen monoklonalen Antikörper, der an den auf den Lymphozyten befindlichen menschlichen $IL_2$-Rezeptor binden kann und in vitro, auch in Gegenwart von Komplement, nicht zytotoxisch ist, dabei aber die durch $IL_2$ induzierte Zellvermehrung inhibiert, mit einem pharmazeutisch akzeptablen Exzipienten mischt.

**2.** Verfahren nach Anspruch 1, dadurch gekennzeichnet, daß der Antikörper ein monoklonaler Antikörper der Maus der Klasse IgG1 ist.

**3.** Verfahren nach Anspruch 1, dadurch gekennzeichnet, daß der Antikörper ein monoklonaler Antikörper der Ratte der Klasse IgG2a ist.

**4.** Verfahren nach Anspruch 1, dadurch gekennzeichnet, daß der Antikörper ein menschlicher monoklonaler Antikörper ist.

**5.** Verfahren nach Anspruch 1, dadurch gekennzeichnet, daß der Antikörper ein monoklonaler Antikörper menschlichen oder tierischen Ursprungs ist, der chemisch modifiziert wurde, um die zytotoxischen Wirkungen in Gegenwart von Komplement zu unterdrücken.

**6.** Verfahren nach Anspruch 1, dadurch gekennzeichnet, daß der Antikörper menschlichen oder tierischen Ursprungs mit Polyethylenglycol chemisch modifiziert wird.

**7.** Verwendung eines monoklonalen Antikörpers, der an den auf den Lymphozyten befindlichen menschlichen $IL_2$-Rezeptor binden kann und in vitro, auch in Gegenwart von Komplement, nicht zytotoxisch ist, dabei aber die durch $IL_2$ induzierte Zellvermehrung inhibiert, zur Herstellung eines zur Bekämpfung der Abstoßung von Organtransplantaten bestimmten Medikaments.

**8.** Verwendung eines monoklonalen Antikörpers nach nach Anspruch 3 zur Herstellung eines zur Bekämpfung der Abstoßung von Organtransplantaten bestimmten Medikaments.

**9.** Verwendung eines monoklonalen Antikörpers nach einem der Ansprüche 2 und 4 bis 6 zur Herstellung eines zur Bekämpfung der Abstoßung von Organtransplantaten bestimmten Medikaments.

13